(19) Europäisches Patentamt
European Patent Office
Office européen des brevets

(11) Publication number: **0 157 828 B1**

(12) # EUROPEAN PATENT SPECIFICATION

(45) Date of publication of patent specification: **12.01.94** (51) Int. Cl.5: **C07C 269/08**

(21) Application number: **84903579.5**

(22) Date of filing: **11.09.84**

(86) International application number:
**PCT/US84/01437**

(87) International publication number:
**WO 85/01285 (28.03.85 85/08)**

(54) **PROCESS FOR THE PREPARATION OF URETHANES.**

(30) Priority: **16.09.83 US 532784**
**16.09.83 US 532785**

(43) Date of publication of application:
**16.10.85 Bulletin 85/42**

(45) Publication of the grant of the patent:
**12.01.94 Bulletin 94/02**

(84) Designated Contracting States:
**AT BE CH DE FR GB LI LU NL SE**

(56) References cited:
**EP-A- 86 281**
**JP-A- 557 227**
**US-A- 3 448 140**
**US-A- 3 993 685**
**US-A- 4 297 501**

(73) Proprietor: **CATALYTICA ASSOCIATES**
**430 Ferguson Drive**
**Building 3**
**Mountain View, CA 94043(US)**

Proprietor: **Haldor Topsoe A/S**
**Nymollevej 55**
**DK-2800 Lyngby(DK)**

(72) Inventor: **GRATE, John, H.**
**1593 Lloyd Way**
**Mountain View, CA 99040(US)**
Inventor: **HAMM, David, R.**
**1033 Crestview Drive, No. 306**
**Mountain View, CA 99040(US)**
Inventor: **VALENTINE, Donald, H.**
**20 Blue Ridge Road**
**Ridgefield, CT 06877(US)**

(74) Representative: **Lehmann, Klaus, Dipl.-Ing. et al**
**Patentanwälte Schroeter & Lehmann,**
**Postfach 71 03 50**
**D-81453 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 157 828 B1

## Description

BACKGROUND OF THE INVENTION

1. Field of the Invention

This invention relates to a process for preparing urethanes by reacting a solution of a nitrogen-containing organic compound and a hydroxyl-containing organic compound with carbon monoxide in the presence of a ruthenium catalyst and a primary amine.

2. Description of the Art

Isocyanates such as toluene diisocyanate (TDI) and 4,4'-diisocyanato diphenyl methane (MDI) are used commercially in the preparation of urethane polymers. The present commercial technology for the preparation of these isocyanates utilizes phosgene, which is costly, toxic, corrosive, and difficult to handle. It is thus understandable that a great deal of recent research has been directed toward different methods for preparing isocyanates, especially TDI and MDI.

Various patents have disclosed methods for carbonylating nitrogen-containing organic compounds, e.g. nitro compounds, amines, azo-and azoxy compounds to either isocyanates or urethanes in the presence of a platinum group metal-containing catalyst; usually a palladium or rhodium-containing catalyst, and most often a palladium halide-containing catalyst. (The urethanes can be decomposed to yield the corresponding isocyanates.) Generally, a cocatalyst (promoter) or a coreactant has been utilized in combination with the aforementioned platinum group metal-containing catalysts; Lewis acids, Lewis bases, oxidizing agents, reducing agents, etc. have been used as cocatalysts or coreactants in the platinum group metal-catalyzed carbonylation of nitrogen-containing organic compounds. It is important to note that the vast majority of the research on the carbonylation of nitrogen-containing organic compounds has been directed to catalysis by rhodium or palladium-containing catalysts; especially palladium halide-containing catalysts. Therefore the cocatalysts or coreactants, that have been disclosed, have a demonstrable effect on the activity and selectivity of a palladium-containing catalyst; but the effect of such cocatalysts on the activity or selectivity of other platinum group metals or compounds is speculative.

Due to the complex nature of catalysis, it is often difficult to predict the effect of a known cocatalyst or coreactant on a catalyst having a different metal as the catalytically active moiety. Therefore, although U. S. Patent 4,178,455 discloses that the reaction rate and yield (in a platinum group metal catalyzed process for preparing an aromatic urethane) is increased by a promoter consisting of a Lewis acid (e.g. metal halides, and especially iron chlorides) and an organic primary amino compound, a urea compound, a biuret compound, an allophanate compound, or a mixture thereof, it is not obvious that such promoter is effective either in the absence of the Lewis acid or with a platinum group metal or platinum group metal compound other than palladium or palladium chloride. As a result, the effect of the promoter, disclosed in U. S. Patent 4,178,455, on other catalyst metals can not be predicted with a reasonable degree of certainty.

In the few references which suggest that ruthenium compounds are suitable catalysts for the carbonylation of nitrogen-containing organic compounds to the corresponding urethanes or isocyanates, the catalyst is either a ruthenium halide, or a halide-containing moiety is combined with the ruthenium compound to provide the active catalyst. For example, in U. S. Patents 3,660,458; 4,134,880; and 4,186,269; the ruthenium compound that has demonstrated catalytic activity is ruthenium chloride. In U. S. Patents 3,461,149 and 3,979,427 ruthenium-on-alumina is treated with halide-containing compounds, such as ferric chloride or 1,1,2-trichloro - 1,2,2,-trifluoroethane, to provide a heterogeneous catalyst.

Another example of a heterogeneous ruthenium catalyst for the preparation of aromatic isocyanates may be found in U.S. Patent 3,737,445. This patent discloses a gas-phase process for reacting carbon monoxide with an aromatic nitro or nitroso compound to yield an aromatic isocyanate.

It is also well knownthat the ligand or anion associated with a platinum group metal will vary the catalytic properties thereof. In a process for manufacturing urethanes from alcohols and phenols, carbon monoxide and nitro compounds, in the presence of a catalyst comprising a transition metal complex, as disclosed in U. S. Patent 3,448,140, the presence of a chelating bis phsphino moiety increases the yield of iridium-containing complexes and decreases the yield of rhodium-containing complexes. (Compare Example Nos. 1 and 2 with Example Nos. 4 and 5). Therefore, although the combination of a phosphine ligand with a platinum group metal catalyst moiety is suggested in U. S. Patents 3,454,620; 3,523,962; and 3,993,685 (as well as U. S. Patent 3,448,140); there is no basis for predicting the behavior of a catalyst comprising the combination of phosphine ligands and a ruthenium moiety, from the demonstrated catalytic behavior of

phosphine ligands in combination with other platinum group metal compounds.

Ruthenium compounds have been utilized in the reduction of organic nitro compounds to the corresponding amines with mixtures of hydrogen and carbon monoxide. It was reported in U. S. 3,729,512 that the reduction of the organic nitro compound with carbon monoxide and ethanol, in the absence of $H_2$, resulted in a mixture of amine and a urethane. The patentee was not concerned with the preparation of a urethane product; therefore, there was no attempt to increase the selectivity above the approximately 22 percent, urethane, that was obtained.

US-4,297,501 relates to the oxycarbonylation of primary amines with carbon monoxide and hydroxyl-containing compounds in the presence of molecular oxygen and/or organic nitro compounds as oxidizing agents and in the presence of certain catalysts. Ruthenium catalysts are not explicitly mentioned.

## SUMMARY OF THE INVENTION

It is, accordingly, one object of this invention to provide an improved process for converting a nitrogen-containing organic compound, selected from the group consisting of nitro, nitroso, azo and azoxy compounds into the corresponding urethane by reacting a solution, comprising a hydroxyl-containing organic compound and the nitrogen-containing organic compound, with carbon monoxide, in the presence of a ruthenium-containing catalyst.

Subject matter of the invention is a process for converting a nitrogen-containing organio compound, selected from the group of nitro, nitroso, azo and azoxy compounds, into the corresponding urethane, by reacting a solution, comprising said nitrogen-containing organic compound and a hydroxyl-containing organic compound, with carbon monoxide, which comprises the steps of:

(a) providing a primary amine in said solution, and

(b) contacting the solution of step (a) with carbon monoxide, in the presence of a catalyst comprising a ruthenium compound, at a temperature of from 80 to 230 °C and at an initial carbon monoxide pressure in the range of from 10 to 1,000 kg/cm$^2$ G.

Preferred embodiments of the invention are as defined claims 2 to 23.

While not wishing to be bound by theory, it appears that, in the ruthenium catalyzed carbonylation of the above nitrogen-containing organic compound to the corresponding urethane, the nitrogen-containing organic compound must first be reduced to a primary amine which then undergoes oxidative carbonylation to the urethane. These reactions which are illustrated below (wherein [H] represents the ruthenium hydrogen carrier) must be effectively coupled to provide the desired selectivity to the urethane.

Oxidative carbonylation: $C_6H_5NH_2 + CO + CH_3OH \rightarrow C_6H_5NHCO_2CH_3 + 2[H]$

Reduction/hydrogenation: $C_6H_5NO_2 + 2CO + 2[H] \rightarrow C_6H_5NH_2 + 2CO_2$

Net reaction: $C_6H_5NO_2 + 3CO + CH_3OH \rightarrow C_6H_5NHCO_2CH_3 + 2CO_2$

Thus the primary amine (illustrated by aniline) is an intermediate in the formation of urethane from the nitrogen-containing organic compound. It has been found that the halide-free ruthenium compounds used as catalysts in this invention are able to efficiently and rapidly reduce the nitrogen-containing organic compounds to the primary amine. The presence of iron chlorides or similar Lewis Acids is ineffective for increasing the activity of halide-free ruthenium catalysts.

The primary amine may also be provided by the in-situ decomposition of a urea or a biuret compound to the corresponding primary amine (s) and urethane in the reaction solution. It has been found that the primary amine substantially increases the rate of conversion of the nitrogen-containing organic compound to the corresponding urethane.

Moreover, it has been found that the combination of a halide-free bisphosphine ruthenium catalyst and a primary amine substantially increases the rate of conversion of the nitrogen-containing organic compound to the corresponding urethane and provides a selectivity to urethane of 88 percent, or greater, at 100 percent conversion of the nitrogen-containing organic compound.

DETAILED DESCRIPTION OF THE INVENTION

The nitrogen-containing organic compound useful in the process of this invention will contain at least one non-cyclic group in which a nitrogen atom is directly attached to a single carbon atom and through a double bond to oxygen or another nitrogen atom. The nitrogen-containing organic compound is selected from the group consisting of nitro, nitroso, azo and azoxy compounds.

Examples of suitable nitrogen-containing organic compounds for use in the process of this invention are compounds represented by the general formulae:

I $R_1\ (NO_x)_y$ and

II $R_1\text{-}N=N\ (O)_z\ \text{-}\ R_2$

wherein $R_1$ and $R_2$ are radicals independently selected from the group consisting of $C_1$ to $C_{20}$ hydrocarbyl radicals and substituted derivatives thereof, x is an integer of from 1 to 2, y is an integer of from 1 to 3, and z is an integer of from 0 to 1. The substituted hydrocarbyl radical may include hetero atoms selected from the group consisting of halogen, oxygen, sulfur, nitrogen and phosphorous atoms.

The nitrogen-containing compounds represented by formula I include nitro compounds (wherein x is 2) and nitroso compounds (wherein x is 1). Suitable nitro compounds are mononitro compounds such as nitrobenzene, alkyl and alkoxy nitrobenzenes wherein the alkyl group contains up to 10 carbon atoms, aryl and aryloxy nitrobenzenes, wherein the aryl group is phenyl, tolyl, naphthyl, xylyl, chlorophenyl, chloronitrobenzenes, aminonitrobenzenes, carboalkoxyamino nitrobenzenes wherein the alkoxy group has up to 10 carbon atoms, aryl and aryloxy dinitrobenzenes, trinitro compounds such as trinitrobenzene, alkyl and alkoxytrinitrobenzenes, aryl and aryloxytrinitrobenzenes, the substituents being any of those already mentioned and chlorotrinitrobenzenes as well as similarly substituted mono and polynitro derivatives of the naphthalene, diphenyl, diphenylmethane, anthracene and phenanthrene series. Substituted or unsubstituted aliphatic nitro compounds such as nitromethane, nitrobutane, 2,2' -dimethyl nitrobutane, nitrocyclopentane, 3-methylnitrobutane, nitrooctadecane, 3-nitropropene-1, phenyl nitromethane, p-bromophenyl nitromethane, p-methoxy phenyl nitromethane, dinitroethane, dinitrohexane, dinitrocyclohexane, di-(nitrocyclohexyl)-methane are also suitable. The above nitro compounds may include more than one of the above substitutents (in addition to the nitro group (s) such as in nitroaminoalxylbenzenes, nitroalkylcarboalkoxyamino benzenes, etc. From this group of nitro compounds nitrobenzene, nitrotoluene, dinitrobenzene, dinitrotoluene, trinitrobenzene, trinitrotoluene, mononitronaphythalene, dinitronaphthalene, 4,4'-dinitrodiphenylmethane, nitrobutane, nitrocyclohexane, p-nitrophenylnitromethane, dinitrocyclohexane, dinitromethylcyclohexane, dinitrocyclohexylmethane, nitroaminotoluene and nitrocarboalkoxyaminotoluene are preferred and in particular aromatic nitro compounds especially 2,4-and 2,6-dinitrotoluenes, meta and para dinitrobenzenes, and 5-nitro-2-methyl-carboalkoxyamino-, 2-nitro-5-methyl-carboalkoxyamino-, and 3-nitro-2-methyl-carboalkoxyamino benzenes.

Examples of suitable nitroso compounds are the aromatic nitroso compounds such as nitrosobenzene, nitrosotoluene, dinitrosobenzene, dinitrosotoluene and the aliphatic nitroso compounds such as nitrosobutane, nitrosocyclohexane and dinitrosomethylcyclohexane.

The nitrogen-containing compounds represented by Formula II include both azo compounds (wherein z is 0) and azoxy compounds (wherein z is 1). Suitable compounds represented by formula II include azobenzene, nitroazobenzene,chloroazobenzene,alkyl or aryl substituted azobenzene, azoxybenzene, nitroazoxybenzene, chloroazoxybenzene, etc.

The hydroxy-containing organic compounds for use in the process of this invention include compounds represented by the general formula

III $R_1\ (OH)_y$

wherein $R_1$ and y are defined above.

Hydroxy compounds suitable for use in the process of the present invention may be, for example, mono-or polyhydric alcohols containing primary, secondary or tertiary hydroxyl groups as well as mono-and polyhydric phenols. Mixtures of these hydroxy compounds may also be used. The alcohols may be aliphatic or aromatic and may bear other substituents in addition to hydroxyl groups but the substituents should (except as hereinafter described) preferably be non-reactive to carbon monoxide under the reaction conditions. Especially suitable compounds are phenol and monohydric alcohols such as methyl, ethyl, n-and sec-propyl, n-, iso, sec-and tert butyl, amyl, hexyl, lauryl, cetyl, benzyl, chlorobenzyl and methoxybenzyl alcohols as well as diols such as ethylene glycol, diethylene glycol, propylene glycol and dipropylene glycol, triols such as glycerol, trimethylol propane, hexanetriol, tetrols such as pentaerythritol and the ethers of such polyols providing that at least one hydroxyl group remains unetherified. The etherifying group in such ether alcohols normally contains up to 10 carbon atoms and is preferably an alkyl, cycloalkyl or aralkyl group which may be substituted with, for example, a halogen or an alkyl group.

The most preferred hydroxyl-containing organic compound for use in the process of this invention is methyl alcohol or a similar lower alkanol, e.g. a $C_1$ to $C_5$ alcohol.

The process of this invention includes the use of any mixture of nitro compounds, nitroso compounds, azo or azoxy compounds with any mixture of hydroxy compounds and also the use of compounds containing both functions, i.e. hydroxynitro compounds, hydroxynitroso compounds, hydroxyazo and hydroxyazoxy compounds such as 2- hydroxynitroethane, 2-hydroxynitrosoethane, nitrophenols, nitronaphthols, nitrosophenols, nitrosonaphthols, hydroxyazobenzenes and hydroxyazoxybenzenes. Mixtures of these nitrogen-containing compounds may also be used.

This process of the invention has been found to proceed most smoothly to give the highest yields when employing nitro compounds. It is accordinly preferred to use nitro compounds rather than nitroso, azo or azoxy compounds.

The primary amine compound utilized in the process of this invention may be selected from the group consisitng of compounds represented by the general formula:

IV $R_1\ (NH_2)_Y$

wherein $R_1$ and Y are as defined above. Examples of such primary amines include methylamine, ethylamine, butylamine, hexylamine, ethylenediamine, propylenediamine, butylenediamine, cyclohexylamine, cyclohexyldiamine, aniline, p-toluidine, o-, m-and p-diaminobenzenes, amino-methylcarbanilic acid esters, especially the 5-amino-2 methyl-, 2-amino-5-methyl-, and 3-amino-2-methyl carboalkoxyaminobenzenes, wherein said alkoxy group has up to 10 carbon atoms, o-, m- and p-nitroanilines, nitroaminotoluenes, especially those designated above, o-and p- phenylenediamine, benzylamine, o-amino-p-xylene, 1-aminophthaline, 2,4-and 2,6-diaminotoluenes, 4,4'-diaminodibenzyl, bis (4-aminophenyl) thioether, bis (4-aminophenyl) sulfone, 2,4,6-triaminotoluene, o-, m-and p-chloranilines, p-bromoaniline, 1-fluoro-2,4-diaminobenzene, 2,-4-diaminophenetole, o,-m- and p-aminoanisoles, ethyl p-aminobenzoate, 3-aminophthalic anhydride, etc. These amino compounds may be used alone or in combination.

Among the above-enumerated amino compounds, those which can be derived from the starting nitro compound are preferred. For example, when nitrobenzone is used as the starting aromatic nitro compound, aniline is preferred. Similarly, 2-amino-4-nitrotoluene, 4-amino-2-nitrotoluene, and 2,4-diaminotoluene are preferably used when the starting aromatic nitro compound is 2,4-dinitrotoluene, while 2-amino-6-nitrotoluene, and 2,6-diaminotoluene are preferably used when the starting aromatic nitro compound is 2,6-dinitrotoluene.

The primary amine compound can be provided by the in-situ decomposition of the corresponding urea or biuret as represented by compounds having the general formulae:

$$R_1NH - \underset{\displaystyle O}{C} - NHR_1$$

and

$$R_1NH - \underset{\displaystyle O}{C} - \underset{\displaystyle R_1}{N} - \underset{\displaystyle O}{C} - NHR_1$$

respectively, wherein $R_1$ is as defined above. Of course, since the above urea and biuret will comprise more than one radical, $R_1$ may represent different radicals in the same compound. That is non-symmetrical ureas and biurets, e.g.

$$CH_3NH\underset{\displaystyle O}{C} - NHC_2H_5$$

are within the scope of the invention.

In one embodiment, the catalyst utilized in the process of this invention comprises a halide-free ruthenium compound. Unlike other platinum group metal-containing catalysts for the carbonylation of nitrogen-containing organic compounds, the presence of halide in ruthenium catalysts, either as the anion of a ruthenium salt or in a Lewis acid decreases the activity of the ruthenium catalyst. Thus the rutheniun compound may be selected from ruthenium salts, e.g. the nitrate, sulfate, acetate, formate, carbonate, etc. and ruthenium complexes (especially ruthenium carbonyl complexes) including ligands capable of coordinating with the ruthenium atom. The complex may include one or more ruthenium atoms and suitable ligands may include carbon-carbon unsaturated groups as in ethylene, isobutylene, cyclohexene, cyclopentadiene, norbornadiene, cyclooctatetraene. Other suitable ligands include acetylacetonate (acac), hydrogen atoms, carbon monoxide, nitric oxide, alkylradicals, alkyl or aryl nitriles or isonitriles, nitrogen-containing heterocyclic compounds such as pyridine, 2,2'-bipyridine (bipy), piperidine, and organo phosphines, arsines or stibines.

The ruthenium catalyst is preferably utilized as a homogeneous catalyst and therefore one criteria for the selection of the rutenium compound in its solubility under the conditions of reaction in the mixture of the nitrogen-containing organic compound, the hydroxyl-containing organic compound and the primary amino compound. The ruthenium compound is also selected with a view toward the catalytic activity of the compound. Thus the organo phosphines are useful ligands for incorporation into the ruthenium catalyst utilized in the process of the instant invention.

Suitable organophosphine include compounds represented by the following formula:

V $(R_3)(R_4)P(R_5)$

wherein $R_3$,$R_4$ and $R_5$ are radicals independently selected from the group consisting of hydrogen, hydrocarbyl, and substituted derivatives of hydrocarbyl radicals, and wherein the substituted hydrocarbyl radicals may include heteroatoms selected from the group consisting of halogen, oxygen, sulfur, nitrogen and phosphorous atoms. Preferably the above hydrocarbyl radicals will comprise from 1 to about 20 carbon atoms, e.g. from about 1 to about 10 carbon atoms. Suitable radicals include methyl, ethyl, n-propyl, isopropyl, butyl, 2-chlorobutyl, n-propoxy, 2-nitro pentyl, phenyl, fluorophenyl, o,m, and p-methylphenyl, etc.

Examples of suitable organophosphines include triphenylphosphine, methyldiphenylphosphine, tris o-chlorophenylphosphine, tri-n-propylphosphine, tris-p-methoxybenzylphosphine, etc.

Examples of halide-free ruthenium compounds which are suitable as catalysts for the process of this invention include:

$Ru_3(CO)_{12}$
$H_4Ru_4(CO)_{12}$

Ruthenium acetylacetonate

$Ru_3(CO)_9[P(PC_6H_5)_3]_3$

In another embodiment of this invention the catalyst comprises a bis-phosphino ruthenium compound, i.e. a compound having ruthenium coordinated with at least one bis-phosphino ligand. For the reasons given above the bis-phosphino ruthenium compound, utilized as the catalyst in this embodiment of the present invention is also preferably free of halide.

The bis-phosphino ruthenium compound may also include the anions and/or the other ligands discussed above.

The bis-phosphino ligand of the ruthenium catalyst may be represented by the general formula:

$(R_3)(R_4)P\text{-}R_6\text{-}P(R_3)(R_4)$

wherein $R_3$ and $R_4$ are defined above and $R_6$ is a divalent radical providing sufficient spacing to enable both phosphorous atoms to coordinate with a ruthenium atom. Preferably, $R_5$ comprises from 2 to 6 carbon atoms.

Examples of suitable bis-phosphine ligands include bis (1,2-diphenylphosphino)benzene,
bis(1,2-diphenylphosphino)ethane,
bis(1,3-diphenylphosphino)propane, etc.

The bisphosphino ruthenium catalyst may be preformed or formed in-situ in the reaction solution by separately dissolving a bisphosphino-free ruthenium compound and a bisphosphine. Since the bisphosphino ruthenium compound is utilized in very low concentration, it is preferred that the bisphosphino ruthenium compound is preformed to ensure that the bisphosphino ligand will be coordinated with the ruthenium atom during the reaction.

In the process of this invention, no particular limitation is placed on the amount of primary amine used. However, it is preferably used in an amount equal to from 0.1 to 100 moles per gm-atom of nitrogen in the nitrogen-containing organic compound.

The process of the invention may be carried out in the absence of solvent but the use of a solvent is not precluded. Suitable solvents include, for example, aromatic solvents such as benzene, toluene, xylene, etc.; nitriles such as acetonitrile, benzonitrile, etc.; sulfones such as sulfolane, etc.; halogenated aliphatic hydrocarbons such as 1,1,2-trichloro-1,2,2,-trifluoroethane, etc.; halogenated aromatic hydrocarbons such as monochlorobenzene, dichlorobenzene, trichlorobenzene, etc.; ketones; esters; and other solvents such as tetrahydrofuran, 1,4-dioxane, 1,2-dimethoxyethane, etc.

In carrying out the process of the invention, the hydroxyl-containing organic compound and carbon monoxide may be used in amounts equal to at least 1 mole per gm-atom of nitrogen in the nitrogen-containing compound. Preferably the hydroxyl-containing orgainic compaound is used in excess and functions as a solvent as well as reactant.

The amount of the ruthenium compound used as the catalyst may vary widely according to the type thereof and other reaction conditions. However, on a weight basis, the amount of catalyst is generally in the range of from $1 \times 10^{-5}$
to 1 part, and preferably from $1 \times 10^{-4}$ to $5 \times 10^{-1}$ parts, per gram-atom of nitrogen in the starting nitrogen-containing organic compound when expressed in terms of its metallic component.

The reaction temperature is generally held in the range of 80° to 230° C., and preferably in the range of from 130° to 200° C.

The reaction pressure, or the initial carbon monoxide pressure, is generally in the range of from 10 to 1,000 kg/cm$^2$G, and preferably from 30 to 500 kg/cm$^2$G.

The reaction time depends on the nature and amount of the nitrogen-containing organic compound used, the reaction temperature, the reaction pressure, the type and amount of catalyst used, the type of reactor employed, and the like, but is generally in the range of from 5 minutes to 6 hours. After completion of the reaction, the reaction mixture is cooled and the gas is discharged from the procedure including filtration, distillation, or other - suitable separation steps, whereby the resulting urethane is separated from any unreacted materials, any by-products, the solvent, the catalyst, and the like.

The urethanes prepared by the process of the invention have wide applications in the manufacture of agricultural chemicals, isocyanates, and polyurethanes.

The invention is more fully illustrated by the following examples.

In each of the following examples, the reaction was conducted in batch mode in a 300 ml stainless steel autoclave reactor equipped with a stirring mechanism which provides constant dispersion of the gas through the liquid solution. Heating of the reaction is provided by a jacket-type furnace controlled by a proportioning controller. The autoclave is equipped with a high pressure sampling system for removal of small samples of the reaction solution during the reaction in order to monitor the reaction progress and determine reaction rates. Reaction samples were analyzed by gas chromatography.

EXAMPLE 1

75 ml of solution containing 6.16 g (0.050 mole) of nitrobenzene, 4.66 g (0.050 mole) of aniline, and 2.68 g of t-butylbenzene (internal standard for gas chromatographic analyses) in methanol and 0.128 g $Ru_3(CO)_{12}$ (600 microgram-atoms of ruthenium) were placed in the reactor vessel. The gas volume in the vessel was replaced with carbon monoxide and then pressurized with carbon monoxide to 69 bar (1000 psig) at ambient temperature. The reactor contents were then heated to 160° C. The initial turnover frequency at this temperature was determined to be 0.36 moles nitrobenzene converted per gram-atom ruthenium per minute. After 4.5 hours at 160° C., nitrobenzene conversion was complete and the solution contained 0.037 mole methyl-N-phenyl carbamate (74% selectivity based on nitrobenzene), 0.059 mole aniline (18% selectivity based on nitrobenzene), and 0.003 mole total methyl-N-phenyl carbamate (74% selectivity based on nitrobenzene), 0.059 mole aniline (18% selectivity based on nitrobenzene), and 0.003 mole total of formylidene aniline plus N-methyl aniline (6% selectivity).

EXAMPLE 2

The procedure is the same as for Example 1 with the exception that no aniline is introduced to the reaction. Additional methanol was added so that the total initial solution volume is again 75 ml. The initial turnover frequency at 160° C. is 0.10 moles nitrobenzene converted per gram-atom ruthenium per minute. Complete nitrobenzene conversion requires 12 hours at 160° C. Selectivities based on nitrobenzene are 24 percent to phenyl urethane (methyl-N-phenyl carbamate), 58 percent to aniline, and 11 percent total to formylidene aniline plus N-methyl aniline. The balance is converted to higher molecular weight products derived from aniline.

It is thus clear that the rate of conversion of nitrobenzene and the selectivity of the conversion of nitrobenzene to the corresponding urethane is increased by providing the corresponding primary amine in the reaction solution.

EXAMPLES 3-5

For these examples, the procedure is the same as for Example 1 with the exception that various halide-free ruthenium compounds and various ruthenium catalyst loadings are used. The results obtained are given in Table 1.

TABLE 1

| Example | Ruthenium Compound | Catalyst Loading (microgram-atom Ru) | Initial Turnover Frequency at 160° C. $(min^{1})$ | Selectivity to Phenyl-Urethane at 100% Nitrobenzene Conversion |
|---|---|---|---|---|
| 3 | $Ru_3(CO)_{12}$ | 60 | 0.65 | 74 |
| 4 | $H_4Ru_4(CO)_{12}$ | 600 | 0.40 | 78 |
| 5 | $(Ru(CO)_2(HCO_2))_n$ n is an integer | 200 | 0.48 | 80 |
| 6 | $Ru_3(CO)_9(P(C_6H_5)_3)_3$ | 600 | 0.36 | 72 |
| 7 | $(Ru(CO)_2(HCO_2)(P(c\text{-}C_6H_{11})_3))_2$ | 200 | 0.46 | 80 |

EXAMPLE 8

The procedure is the same as for Example 1 with the exception that 0.24 g tris (acetylacetonate) ruthenium (600 ug-atoms ruthenium) is used as a catalyst precursor. After an initial induction period, the rate of nitrobenzene conversion reaches 0.47 moles nitrobenzene per g-atom ruthenium per minute. After 6

hours at 160° C., nitrobenzene conversion is complete. Selectivities based on nitrobenzene are 65 percent to phenylurethane, 26 percent to aniline, and 9 percent to formylidene aniline. This example demonstrates that ruthenium compounds, that do not contain carbon monoxide ligands, are useful as catalysts in the process of this invention; however, an induction period may be necessary to convert such ruthenium compounds to the carbonyl.

EXAMPLE 9

The procedure is the same as for Example 1 with the exception that 0.13 g ruthenium trichloride (630 ug-atoms ruthenium) is introduced as catalyst. After 5 hours at 160° C., nitrobenzene conversion is 7 percent with 100 percent selectivity to aniline. Complete nitrobenzene conversion requires 24 hours and gives 38 percent selectivity to phenyl urethane and 21 percent selectivity to aniline. The balance is converted to higher molecular weight materials.

EXAMPLE 10

The procedure is the same as for Example 1 with the exception that 0.154 g $[Ru(CO)_3Cl_2]_2$ (600 ug-atoms ruthenium) is introduced as catalyst. After 5 hours at 160° C., nitrobenzene conversion was 8 percent with 100 percent selectivity to aniline.

EXAMPLE 11

The procedure is the same as for Example 1 with the exception that both 0.128 g $Ru_3(CO)_{12}$ and 0.126 g $RuCl_3$ to phenyl urethane is 47 percent. Selectivity to aniline is 53 percent. This example demonstrates that halide salts, even ruthenium halides, can adversely affect the conversion and selectivity of the otherwise effective catalyst of Example 1.

EXAMPLE 12

The procedure is the same as for Example 1 with the exception that both 0.043 g $Ru_3(CO)_{12}$ and 0.036 g $PdCl_2$ are introduced to the reaction. After 5 hours reaction at 160° C., nitrobenzene conversion is 10 percent. Selectivity to phenyl urethane is 5 percent. Selectivity to aniline is 95 percent. This example demonstrates the difference between the prior art palladium catalyst and the catalysts used in the process of this invention. In particular, it would appear that the mode of action of the ruthenium catalysts described herein and the prior art palladium catalyst differ significantly in that the individual catalytic effectiveness of each is hindered by combination with the other.

EXAMPLE 13

The procedure is the same as for Example 1 with the exception that the initial carbon monoxide pressure at ambient temperature is 34,5 bar (500 psig). Nitrobenzene conversion was complete after 6 hours at 160° C. and selectivity to phenyl urethane is 68 percent. This example demonstrates that a lower partial pressure of carbon monoxide may be used, in the process of this invention, with only a slight decrease in rate of reaction and selectivity to urethane.

EXAMPLE 14

The procedure was the same as for Example 1 with the exception that 5.36g (0.050 mole) of p-toluidine was used instead of aniline. After 3 hours at 160° C, nitrobenzene conversion was complete and the solution contained 0.013 mole methyl-N-phenyl carbamate, 0.031 mole methyl-N-(tolyl) carbamate, 0.037 mole aniline and 0.019 mole p-toluidine. nitrobenzene conversion was complete and the solution contained 0.013 mole methyl-N-phenyl carbamate, 0.031 mole methyl-N-(tolyl) carbamate, 0.037 mole aniline and 0.019 mole p-toluidine. This example shows that when the primary amine does not correspond to the nitro compound, mixtures of urethanes may be obtained.

EXAMPLE 15

The procedure is the same as for Example 1 with the exception that 6.86 g p-nitrotoluene and 5.36 g p-toluidine are used as the nitroaromatic and arylamine. Nitrotoluene conversion is 100 percent after 3 hours at 160° C. Selectivity to methyl N-p-tolylcarbamate is 88 percent. Selectivity to p-toluidine is 10 percent. This example demonstrates that certain primary amines are more efficient in increasing the rate of reaction than others. For example, substituents such as a p-$CH_3$, having a σp of -0.17, or substituents having a more negative Hammet σ value, increase the rate of conversion of the nitroaromatic compound.

EXAMPLE 16

6.16g(0.050 mole) nitrobenzene and 10.6 g (0.050 mole) N,N'-diphenyl urea were reacted in methanol by the procedure given in Example 1. The catalyst was 0.126 g (200 micromoles) of [bis(1,2-diphenylphosphino)benzene] ruthenium tricarbonyl. As the reaction contents were heated to 160° C. most of the N,N'-diphenyl urea was converted to equal parts aniline and phenyl urethane. The rate of nitrobenzene conversion at 160° C. reached 0.86 moles nitrobenzene converted per mole ruthenium per minute. After 5 hours at 160° C. nitrobenzene conversion was 100 percent and the solution contained 0.092 moles phenyl urethane and 0.048 moles aniline. This example demonstrates that a urea, which decomposes to provide a primary amine, i.e. aniline, in-situ, also increases the rate of conversion of

EXAMPLE 17

12.3 g (0.100 mole nitrobenzene) in 75 ml of methanolic solution and 0.128 g $Ru_3(CO)_{12}$ (600 microgram-atoms ruthenium) were placed in the reactor vessel. The gas volume in the vessel was replaced with carbon monoxide and then pressurized at ambient temperature with 63,5 bar (920 psig) carbon monoxide and 5,5 bar (80 psig) hydrogen (approximately 0.05 mole hydrogen). The reactor contents were then heated to 160° C. Initially, aniline was produced from nitrobenzene in 100 percent selectivity. After approximately 0.030 moles aniline had been produced, phenyl urethane production also began. After a total of 7.5 hours at 160° C., nitrobenzene conversion was complete and the solution contained 0.060 moles aniline and 0.034 moles phenyl urethane. This example demonstrates that the primary amine may be provided by reduction of the nitrogen-containing organic compound, e.g. nitrobenzene, in-situ. Note that, once the added hydrogen is utilized to reduce the nitrobenzene to aniline, the selectivity of the conversion of the remaining nitrobenzene to the urethane is greater than 50 percent.

EXAMPLE 18

12.3 g (0.100 mole) nitrobenzene and 0.90 g (0.050 mole) water in 75 ml methanolic solution and 0.128 g $Ru_3(CO)_{12}$ (600 microgram-atoms ruthenium) were reacted under carbon monoxide as described in Example 1. When the reaction solution reached 160° C., 0.030 moles of aniline were present and phenyl urethane production began. After 6 hours at 160° C., nitrobenzene conversion was complete and the solution contained 0.061 moles aniline and 0.034 moles phenyl urethane. This example demonstrates that water can be utilized to provide hydrogen to reduce the nitrogen-containing organic compound (nitrobenzene) to the primary amine (aniline), in-situ. Again, as in Example 14, once substantially all of the hydrogen equivalents generated by the water-gas shift reaction are utilized to reduce nitrobenzene to aniline, the selectivity of the conversion of the remaining nitrobenzene to the urethane is greater than 50 percent.

EXAMPLE 19

75 ml of solution containing 6.16 g (0.050 moles) of nitrobenzene, 4.66 g (0.050 moles) of aniline, and 2.68 g t-butylbenzene (internal standard for gas chromatographic analysis) in methanol and 0.350 g of [bis-(1,2-diphenylphosphino)ethane] ruthenium tricarbonyl (600 microgramatoms of ruthenium) are placed in the reaction vessel. The gas volume in the vessel is replaced with carbon monoxide and then pressurized to 69 bar (1000 psig) at ambient temperature. The reactor contents are then heated to 160° C. The initial turnover frequency at this temperature is determined to be 0.85 moles nitrobenzene converted per g-atom ruthenium per minute. After 90 minutes at 160° C., nitrobenzene conversion is complete. Selectivity to methyl N-phenyl carbamate is 88 percent based on nitrobenzene converted.

In comparing this example to Example 1 it is discovered that the use of a bisphosphino ruthenium compound as the catalyst for the conversion of the above-defined nitrogen-containing organic compound,

e.g. nitrobenzene, to a urethane, in the presence of a hydroxyl-containing organic compound, e.g. methanol, and a primary amine, results in an increased rate of reaction and selectivity as compared to a bis-phosphine-free ruthenium compound.

EXAMPLES 20 - 30

For these examples, the procedure is the same as for Example 19 with the exceptions that various ruthenium compounds containing Group V ligands are used as catalyst precursors. The results are given in Table 2. compounds containing Group V ligands are used as catalyst precursors. The results are given in Table 2.

TABLE 2

| Example | Ruthenium Compound | Catalyst Loading (microgran-atoms Run) | Initial Turnover Frequency at 160° C. |
|---|---|---|---|
| 20 | [bis(diphenylphosphino ethane] ruthenium tricarbonyl | 60 | 1.2 |
| 21 | [bis(diphenylphosphino)propane]ruthenium tricarbonyl | 60 | 0.87 |
| 22 | [bis(1,2-diphenylphosphino)benzene]ruthenium tricarbonyl | 200 | 0.93 |
| 23 | [bis(1,2-diphenylphosphino)benzene]ruthenium dicarbonyl bis (trifluroacetate) | 200 | 0.44 |
| 24 | [bis(1,2-dimethylphosphino)ethane]ruthenium tricarbonyl | 200 | 0.45 |
| 25 | [bis(1,2-dicyclohexylphosphino)ethane]ruthenium tricarbonyl | 200 | 0.28 |
| 26 | [bis(1,2-dimethylarsino)benzene]ruthenium tricarbonyl | 200 | 0.19 |
| 27 | [bis-(2-diphenylphosphinoethyl)phenylphosphine]ruthenium dicarbonyl | 200 | 0.16 |
| 28 | bis(triphenylphosphine)ruthenium tricarbonyl | 600 | 0.25 |
| 29 | tris(triphenylphosphine)triruthenium nonacarbonyl | 600 | 0.36 |
| 30 | bis(triphenylphosphine)ruthenium dicarbonyl dichloride | 600 | 0.09 |

EXAMPLE 32

The procedure is the same as for Example 19 with the exceptions that 0.063 g (100 micromoles) [bis-(1,2-disphenylphosphino)benzene]ruthenium tricarbonyl is used as catalyst and the reaction temperature is

177° C. The initial turnover frequency at this temperature is 2.8 moles nitrobenzene converted per gram-atom ruthenium per minute. This example also demonstrates that the reactivity of the ruthenium catalyst may be increased (significantly) by increasing the temperature of the reaction.

EXAMPLE 33

The procedure is the same as for Example 22 with the exception that the initial carbon monoxide pressure at ambient temperature is 34,5 bar (500 psig). The initial turnover frequency at 160° C. is 1.1 moles nitrobenzene converted per gram-atom ruthenium per minute. At 100% nitrobenzene conversion, selectivity to phenyl urethane is 85%. This example demonstrates that lower pressures of CO may be used successfully in the process of this invention.

EXAMPLE 34

6.16 g (0.050 mole) nitrobenzene and 10.6 g (0.050 mole) N,N'-diphenyl urea were reacted in methanol by the procedure given in Example 19. The catalyst was 0.126 g (200 micromoles) of [bis(1,2-diphenylphosphino)benzene] ruthenium tricarbonyl. As the reaction contents were heated to 160° C. most of the N,N'-diphenyl urea was converted to equal parts aniline and phenyl urethane. The rate of nitrobenzene conversion at 160°C reached 0.86 moles nitrobenzene converted per mole ruthenium per minute. After 5 hours at 160° C, nitrobenzene conversion was 100% and the solution contained 0.092 moles phenyl urethane and 0.048 moles aniline. This example demonstrates that bis-phosphine containing ruthenium catalysts give an increased rate of reaction when primary amine is formed in the reaction by in-situ decomposition of urea. containing ruthenium catalysts give an increased rate of reaction when primary amine is formed in the reaction by in-situ decomposition of urea.

EXAMPLE 35

The procedure was the same as for Example 19 with the exception that 5.36 g (0.050 mole) p-toluidine was used instead of aniline. The initial turnover frequency at 160° C. was 1.0 moles nitrobenzene converted per g-atom ruthenium per minute. After 70 minutes at 160° C., nitrobenzene conversion was complete and the solution contained 0.014 mole methyl-N-phenyl carbamate, 0.028 mole methyl N-(p-tolyl) carbamate, 0.032 mole aniline, and 0.021 mole p-toluidine. This example shows that when a primary amine is supplied which does not correspond to the nitro compound, mixtures of urethanes may be obtained.

## Claims

**1.** A process for converting a nitrogen-containing organic compound, selected from the group consisting of nitro, nitroso, azo, and azoxy compounds, into the corresponding urethane, by reacting a solution, comprising said nitrogen-containing organic compound and a hydroxyl-containing organic compound, with carbon monoxide, which comprises the steps of:
(a) providing a primary amine in said solution, and
(b) contacting the solution of step (a) with carbon monoxide, in the presence of a catalyst comprising a ruthenium compound, at a temperature of from 80° to 230° C. and an initial carbon monoxide pressure in the range of from 10 to 1,000 $kg/cm^2$ G.

**2.** The process of claim 1 wherein said nitrogen-containing organic compound is a nitro compound.

**3.** The process of claim 2 wherein said nitro compound is an aromatic nitro compound.

**4.** The process of claim 3 wherein said primary amine is an aromatic amine.

**5.** The process of claim 4 wherein said ruthenium compound comprises a phosphine ligand.

**6.** The process of claim 1 wherein said primary amine is provided by reduction of said nitrogen-containing compound with hydrogen in said solution.

**7.** The process of claim 1 wherein said primary amine is provided by reduction of said nitrogen-containing compound with hydrogen equivalents derived from the ruthenium-catalyzed water-gas shift reaction.

8. The process of claim 3 wherein said aromatic nitro-compound is selected from the group consisting of nitrobenzene, nitroanisole, dinitrotoluene, nitromesitylene, bis (4-nitro-phenyl) methane, nitroaminotoluene and nitrocarbonalkoxyaminotoluene.

9. The process of claim 8 wherein said amine is selected from the group consisting of p-toluidine, aniline, diaminotoluene, bis (4-aminophenyl) methane, aminonitrotoluene, and aminomethylcarbonalkoxyaminobenzene.

10. The process of claim 1 wherein said amine is provided by decomposing a urea or biuret in-situ.

11. The process of claim 1 wherein said nitrogen-containing organic compound is converted into the corresponding urethane, by reacting said solution with carbon monoxide at a temperature of at least 130° C. and a carbon monoxide pressure of at least 14 bar (200 psig).

12. A process of claim 1 wherein the ruthenium catalyst comprises a bis-phosphine ligand.

13. The process of claim 12 wherein said nitrogen-containing organic compound is a nitro compound.

14. The process of claim 13 wherein said nitro compound is an aromatic nitro compound.

15. The process of claim 14 wherein said solution comprises a primary amine.

16. The process of claim 15 wherein said primary amine is an aromatic amine.

17. The process of claim 16 wherein said aromatic nitro compound is selected from the group consisting of nitrobenzene, nitroanisole, dinitrotoluene, nitromesitylene, bis (4-nitrophenyl) methane, nitro aminotoluene and nitrocarboalkoxyaminotoluene.

18. The process of claim 12 wherein said bis-phosphine ligand is selected from the group consisting of bis (1,2-diphenylphosphino) ethane, bis (1,3-diphenylphosphino) propane and bis (1,2-diphenylphosphino) benzene.

19. The process of claim 15 wherein said primary amine is provided by reduction of said nitrogen-containing compound with hydrogen in said solution.

20. The process of claim 15 wherein said primary amine is provided by reduction of said nitrogen-containing compound with hydrogen equivalents derived from the ruthenium-catalyzed water-gas shift reaction.

21. The process of claim 15 wherein said amine is selected from the group consisting of p-toluidine, aniline, diaminotoluene, bis (4-aminophenyl) methane aminonitrotoluene, and aminomethylcarbonalkoxyaminobenzene.

22. The process of claim 15 wherein said amine is provided by decomposing a urea or biuret in-situ.

23. The process of claim 12 wherein said nitro-containing organic compound is converted into the corresponding urethane, by reacting said solution with carbon monoxide at a temperature of at least 130° C. and a carbon monoxide pressure of at least 14 bar (200 psig).

## Patentansprüche

1. Verfahren zur Umwandlung einer stickstoffhaltigen organischen Verbindung, die aus der Gruppe der Nitro-, Nitroso-, Azo- und Azoxyverbindungen ausgewählt ist, in das entsprechende Urethan durch Umsetzung einer Lösung, die diese stickstoffhaltige organische Verbindung und eine hydroxylhaltige organische Verbindung enthält, mit Kohlenmonoxid, umfassend folgende Stufen:
   (a) Bereitstellen eines primären Amins in der Lösung und
   (b) Kontaktieren der Lösung von Stufe (a) mit Kohlenmonoxid in Gegenwart eines Katalysators mit einem Gehalt an einer Rutheniumverbindung bei einer Temperatur von 80 - 230°C und einem

anfänglichem Kohlenmonoxid-Überdruck im Bereich von 10 bis 1000 kg/cm$^2$.

2. Verfahren nach Anspruch 1, wobei es sich bei der stickstoffhaltigen organischen Verbindung um eine Nitroverbindung handelt.

3. Verfahren nach Anspruch 2, wobei es sich bei der Nitroverbindung um eine aromatische Nitroverbindung handelt.

4. Verfahren nach Anspruch 3, wobei es sich beim primären Amin um ein aromatisches Amin handelt.

5. Verfahren nach Anspruch 4, wobei die Rutheniumverbindung einen Phosphinliganden umfaßt.

6. Verfahren nach Anspruch 1, wobei das primäre Amin bereitgestellt wird, indem man die stickstoffhaltige organische Verbindung mit Wasserstoff in der Lösung reduziert.

7. Verfahren nach Anspruch 1, wobei das primäre Amin bereitgestellt wird, indem man die stickstoffhaltige Verbindung mit Wasserstoffäquivalenten, die von der durch Ruthenium katalysierten Wasser-Gas-Verschiebungsreaktion abgeleitet sind, reduziert.

8. Verfahren nach Anspruch 3, wobei die aromatische Nitroverbindung aus der Gruppe Nitrobenzol, Nitroanisol, Dinitrotoluol, Nitromesitylen, Bis-(4-nitrophenyl)-methan, Nitroaminotoluol und Nitrocarbonalkoxyaminotoluol ausgewählt wird.

9. Verfahren nach Anspruch 8, wobei das Amin aus der Gruppe p-Toluidin, Anilin, Diaminotoluol, Bis-(4-aminophenyl)-methan, Aminonitrotoluol und Aminomethylcarbonalkoxyaminobenzol ausgewählt wird.

10. Verfahren nach Anspruch 1, wobei das Amin bereitgestellt wird, indem man einen Harnstoff oder Biuret in situ zersetzt.

11. Verfahren nach Anspruch 1, wobei die stickstoffhaltige organische Verbindung in das entsprechende Urethan umgewandelt wird, indem man die Lösung mit Kohlenmonoxid bei einer Temperatur von mindestens 130 °C und einem Kohlenmonoxid-Überdruck von mindestens 14 bar (200 psig) umsetzt.

12. Verfahren nach Anspruch 1, wobei der Rutheniumkatalysator einen Bis-phosphinliganden enthält.

13. Verfahren nach Anspruch 12, wobei des sich bei der stickstoffhaltigen organischen Verbindung um eine Nitroverbindung handelt.

14. Verfahren nach Anspruch 13, wobei es sich bei der Nitroverbindung um eine aromatische Nitroverbindung handelt.

15. Verfahren nach Anspruch 14, wobei die Lösung ein primäres Amin enthält.

16. Verfahren nach Anspruch 15, wobei es sich beim primären Amin um eine aromatisches Amin handelt.

17. Verfahren nach Anspruch 16, wobei die aromatische Nitroverbindung aus der Gruppe Nitrobenzol, Nitroanisol, Di-nitrotoluol, Nitromesitylen, Bis-(4-nitrophenyl)-methan, Nitroaminotoluol und Nitrocarbonalkoxyaminotoluol ausgewählt wird.

18. Verfahren nach Anspruch 12, wobei der Bis-phosphinligand aus der Gruppe Bis-(1,2-diphenylphosphin)-ethan, Bis-(1,3-diphenylphosphin)-propan und Bis-(1,2-diphenylphosphin)-benzol ausgewählt wird.

19. Verfahren nach Anspruch 15, wobei das primäre Amin bereitgestellt wird, indem man die stickstoffhaltige organische Verbindung mit Wasserstoff in der Lösung reduziert.

20. Verfahren nach Anspruch 15, wobei das primäre Amin bereitgestellt wird, indem man die stickstoffhaltige Verbindung mit Wasserstoffäquivalenten, die von einer durch Ruthenium katalysierten Wasser-Gas-Verschiebungsreaktion abgeleitet sind, reduziert.

**21.** Verfahren nach Anspruch 15, wobei das Amin aus der Gruppe p-Toluidin, Anilin, Diaminotoluol, Bis-(4-aminophenyl)-methan, Aminonitrotoluol und Aminomethylcarbonalkoxyaminobenzol ausgewählt wird.

**22.** Verfahren nach Anspruch 15, wobei das Amin bereitgestellt wird, indem man einen Harnstoff oder Biuret in situ zersetzt.

**23.** Verfahren nach Anspruch 12, wobei die stickstoffhaltige organische Verbindung in das entspechende Urethan umgewandelt wird, indem man die Lösung mit Kohlenmonoxid bei einer Temperatur von mindestens 130 °C und einem Kohlenmonoxid-Überdruck von mindestens 14 bar (200 psig) reduziert.

**Revendications**

**1.** Procédé pour convertir un composé organique contenant de l'azote sélectionné à partir d'un groupe consistant en nitrocomposés, composés nitrosés, azoïques et azoxy, dans l'uréthane correspondant, par réaction d'une solution comportant ledit composé organique contenant de l'azote et un composé organique contenant de l'hydroxyle, avec du monoxyde de carbone, qui comprend les étapes suivantes :

(a) prévoir une amine primaire dans ladite solution, et

(b) mettre en contact la solution de l'étape (a) avec du monoxyde de carbone en présence d'un catalyseur comportant un composé de ruthénium, à une température allant de 90 °C à 230 °C et une pression du monoxyde de carbone initiale dans la plage allant de 10 à 1.000 $kg/cm^2$ G.

**2.** Procédé selon la revendication 1, dans lequel ledit composé organique contenant de l'azote est un nitrocomposé.

**3.** Procédé selon la revendication 2, dans lequel ledit nitrocomposé est un nitrocomposé aromatique.

**4.** Procédé selon revendication 3, dans lequel ladite amine primaire est une amine aromatique.

**5.** Procédé selon la revendication 4, dans lequel ledit composé de ruthénium comprend un ligand de phosphine.

**6.** Procédé selon la revendication 1, dans lequel ladite amine primaire est obtenue par réduction dudit composé contenant de l'azote avec de l'hydrogène dans ladite solution.

**7.** Procédé selon la revendication 1, dans lequel ladite amine primaire est obtenue par réduction dudit composé contenant de l'azote avec des équivalents d'hydrogène dérivés à partir de la réaction catalytique eau/gaz. catalysée par le ruthénium.

**8.** Procédé selon la revendication 3, dans lequel ledit nitrocomposé aromatique est sélectionné à partir d'un groupe consistant en nitrobenzène, nitroanisole, dinitrotoluène, nitromésitylène, bis (4-nitro-phényle) méthane, nitroaminotoluène et nitrocarbonalkoxyaminotoluène.

**9.** Procédé selon la revendication 8, dans lequel ladite amine est sélectionnée à partir d'un groupe consistant en p-toluidine, aniline, diaminotoluène, bis (4-aminophényle) méthane, aminonitrotoluène, et aminométhylcarbonalkoxylaminobenzène.

**10.** Procédé selon la revendication 1, dans lequel ladite amine est obtenue par décomposition d'une urée ou d'un biuret in-situ.

**11.** Procédé selon la revendication 1, dans lequel ledit composé organique contenant de l'azote est converti dans l'uréthane correspondant par la réaction de ladite solution avec du monoxyde de carbone à une température d'au moins 130 °C et une pression de monoxyde de carbone d'au moins 14 bar (200 psig).

**12.** Procédé selon la revendication 1, dans lequel le catalyseur de ruthénium comprend un ligand de bis-phosphine.

**13.** Procédé selon la revendication 12, dans lequel ledit composé organique contenant de l'azote est un nitrocomposé.

**14.** Procédé selon la revendication 13, dans lequel ledit nitrocomposé est un nitrocomposé aromatique.

**15.** Procédé selon la revendication 14, dans lequel ladite solution comporte une amine primaire.

**16.** Procédé selon la revendication 15, dans lequel ladite amine primaire est une amine aromatique.

**17.** Procédé selon la revendication 16, dans lequel ledit nitrocomposé aromatique est sélectionné à partir du groupe consistant de nitrobenzène, nitroanisole, dinitrotoluène, nitromésitylène, bis (4-nitrophényle) méthane, nitro aminotoluène et nitrocarbonalkoxyaminotoluène.

**18.** Procédé selon la revendication 12, dans lequel ledit ligand de bis-phosphine est sélectionné à partir d'un groupe consistant en bis (1,2-diphénylphosphino) éthane, bis (1,2)-diphénylphosphino) propane, et bis (1,2-diphénylphosphino) benzène.

**19.** Procédé selon la revendication 15, dans lequel ladite amine primaire est obtenue par réduction dudit composé contenant de l'azote avec de l'hydrogène dans ladite solution.

**20.** Procédé selon la revendication 15, dans lequel ladite amine primaire est obtenue par réduction dudit composé contenant de l'azote avec des équivalents d'hydrogène dérivés à partir de la réaction catalytique eau/gaz catalysée par le ruthénium.

**21.** Procédé selon la revendication 15, dans lequel ladite amine est sélectionnée à partir d'un groupe consistant en p-toluidine, aniline, diaminotoluène, bis (4-aminophényle) méthane, aminonitrotoluène, et aminométhylcarbonalkoxyaminobenzène.

**22.** Procédé selon la revendication 15, dans lequel ladite amine est obtenue par décomposition d'une urée ou d'un biuret in-situ.

**23.** Procédé selon la revendication 12, dans lequel ledit composé organique contenant de l'azote est converti dans l'uréthane correspondant par réaction entre ladite solution avec du monoxyde de carbone à une température d'au moins 130 °C et une pression de monoxyde de carbone d'au moins 14 bar (200 psig).